# EUROPÄISCHE PATENTANMELDUNG

(11) **EP 4 559 397 A1**
(43) Veröffentlichungstag der Anmeldung: **28.05.2025**
(21) Anmeldenummer: 23212063.4
(22) Anmeldetag: 24.11.2023
(51) Int. Cl.: A61B 6/00, A61B 6/03

(54) **MATERIALDEKOMPOSITION BEI DER DUAL-ENERGIE-RÖNTGENBILDGEBUNG**

(71) Anmelder: Siemens Healthineers AG, 91301 Forchheim (DE)
(72) Erfinder: Roser, Philipp, 91052 Erlangen (DE)
(74) Vertreter: Siemens Healthineers Patent Attorneys

(57) **Zusammenfassung**

In einem computerimplementierten Verfahren zur Materialdekomposition bei der Dual-Energie-Röntgenbildgebung werden ein erster Röntgenbilddatensatz (21, 24) entsprechend einem ersten Röntgenstrahlungsenergiespektrum und ein zweiter Röntgenbilddatensatz (22, 25) entsprechend einem zweiten Röntgenstrahlungsenergiespektrums erhalten. Durch Anwendung eines Dekompositionsmoduls (10), welches eine trainierte erste Funktion enthält, auf Eingangsdaten, welche von dem ersten Röntgenbilddatensatz (21, 24) und dem zweiten Röntgenbilddatensatz (22, 25) abhängen, wird wenigstens ein materialspezifischer Bilddatensatz (13, 14) erzeugt.

## Beschreibung

Die vorliegende Erfindung betrifft ein computerimplementiertes Verfahren zur Materialdekomposition bei der Dual-Energie-Röntgenbildgebung, wobei erste Röntgenbilddaten entsprechend einem ersten Röntgenstrahlungsenergiespektrum und zweite Röntgenbilddaten entsprechend einem zweiten Röntgenstrahlungsenergiespektrums erhalten werden. Die Erfindung betrifft ferner ein entsprechendes Verfahren zur Dual-Energie-Röntgenbildgebung, sowie computerimplementierte Trainingsverfahren, eine Datenverarbeitungsvorrichtung und Computerprogrammprodukte.

Bei der Dual-Energie-Röntgenbildgebung (DE-Röntgenbildgebung) werden verschiedene Röntgenbilddaten, beispielsweise zwei verschiedene Röntgenprojektionsbilder oder zwei verschiedene dreidimensionale Volumenrekonstruktionen, mit unterschiedlichen Röntgenstrahlungsenergiespektren, insbesondere mit unterschiedlichen Strahlungsenergien beziehungsweise Strahlungsleistungen, erzeugt. Dabei wird insbesondere von einer Hochenergieaufnahme oder einem Hochenergiescan einerseits und einer Niedrigenergieaufnahme oder einem Niedrigenergiescan andererseits gesprochen. Die unterschiedlichen Röntgenstrahlungsenergiespektren können durch Variation der Betriebsparameter der Röntgenquelle, insbesondere der Betriebsspannung der Röntgenquelle, und/oder Filter im Strahlengang, etwa zur Strahlaufhärtung, realisiert werden. Die Dual-Energie-Röntgenbildgebung kann zur Erzeugung von Röntgenprojektionsbildern eingesetzt werden, aber auch zur Computertomographie (CT), also als DE-CT-Verfahren, oder zur Kegelstrahlcomputertomographie (CBCT), also als DE-CBCT-Verfahren.

Da die Schwächungskoeffizienten verschiedener Materialien, beispielsweise Cerebrospinalflüssigkeit (CSF), Blut und röntgenselektive Kontrastmittel, etwa jod- oder bariumhaltige Kontrastmittel, unter Variation der Strahlungsenergien unterschiedlich stark schwanken, können aus den verschiedenen Röntgenbilddaten gemäß unterschiedlichen Röntgenstrahlungsenergiespektren Bilder beziehungsweise Rekonstruktionen erzeugt werden, in denen ein Material besonders hervorgehoben ist, beispielsweise das Kontrastmittel, sowie weitere Bilder in denen das Material unterdrückt ist. Man spricht dabei von Materialdekomposition. So kann beispielsweise überprüft werden, ob bei einer Intervention eine signifikante Blutung aufgetreten ist. Bei manchen Krankheitsbildern ist die Kontrastmittel-Aufnahme entscheidend, um den Befund einordnen zu können.

Für die Materialdekomposition können Referenzwerte für die Abschwächung von Materialien wie CSF oder Kontrastmittel bei den verschiedenen Röntgenstrahlungsenergiespektren vorgegeben werden. Zusammen mit den tatsächlich gemessenen Abschwächungswerten kann dann im Wesentlichen unter Verwendung von Methoden der linearen Algebra die Materialdekomposition durchgeführt werden. Als Ergebnis liefert die Materialdekomposition dann materialspezifische Bilddaten. Je nach Zielsetzung, Anwendung und je nachdem, welche Materialen getrennt werden sollen, können die materialspezifischen Bilddaten zum Beispiel ein Kontrastbild enthalten, in dem das Kontrastmittel hervorgehoben ist, beziehungsweise eine entsprechende Kontrastrekonstruktion. Dasselbe ist analog für andere Materialien wie Wasser, Fett, und so weiter möglich. Zusätzlich oder alternativ können die materialspezifischen Bilddaten ein virtuelles Nicht-Kontrast-Bild (VNC-Bild, englisch: virtual non-contrast image), enthalten, in denen das Kontrastmittel unterdrückt ist, beziehungsweise eine entsprechende VNC-Rekonstruktion.

Es kann grundsätzlich wünschenswert sein, eine möglichst genaue Materialdekomposition zu erzielen, um nicht nur eine qualitative Unterscheidung der Materialien treffen zu können, sondern auch eine quantitative Analyse durchführen zu können, beispielsweise um die konkrete Kontrastmittelmenge bestimmen zu können. Effekte, welche die Bildqualität einschränken, beinhalten, insbesondere bei CBCT, unter anderem ein vergleichsweise geringes Signal-zu-Rausch-Verhältnis und/oder Kontrast-zu-Rausch-Verhältnis, Streueffekte, Kegelstrahlartefakte, Trunkierung und so weiter.

Es sind heuristische oder empirische Verfahren bekannt, welche die Bildqualität, insbesondere bei CBCT-Verfahren, verbessern können, was dann auch beim Ergebnis der Materialdekomposition zu einer verbesserten Bildqualität führt. Diese verbesserte Bildqualität betrifft jedoch in erster Linie die visuelle Wahrnehmung durch einen menschlichen Betrachter, können die Eignung zur quantitativen Analyse jedoch nicht oder nur unzureichend verbessern.

Zur Qualitätsverbesserung können auch maschinell trainierte Algorithmen eingesetzt werden. Aufgrund ihrer starken Abhängigkeit von den verwendeten Trainingsdaten besteht bei solchen Algorithmen jedoch potentiell das Problem von sogenanntem Overfitting, sodass diese die Eignung zur quantitativen Analyse ebenfalls nicht oder nur eingeschränkt verbessern können.

Es ist eine Aufgabe der vorliegenden Erfindung, die Qualität der materialspezifischen Bilddaten bei der Materialdekomposition bei der Dual-Energie-Röntgenbildgebung zu verbessern, sodass insbesondere eine quantitative Materialanalyse ermöglicht oder eine verbesserte quantitative Materialanalyse ermöglicht wird.

Diese Aufgabe wird durch den Gegenstand des unabhängigen Anspruchs gelöst. Weitere Entwicklungen und bevorzugte Ausführungsformen sind Gegenstand der abhängigen Ansprüche.

Die Erfindung beruht auf der Idee, die Materialdekomposition selbst durch Anwendung einer trainierten Funktion, insbesondere einer durch maschinelles Lernen trainierten Funktion, durchzuführen, deren Eingangsdaten Röntgenbilddatensätze entsprechend unterschiedlicher Röntgenstrahlungsenergiespektren sind.

Gemäß einem Aspekt der Erfindung wird ein computerimplementiertes Verfahren zur Materialdekomposition bei der Dual-Energie-Röntgenbildgebung angegeben. Dabei werden ein erster Röntgenbilddatensatz entsprechend einem ersten Röntgenstrahlungsenergiespektrum und ein zweiter Röntgenbilddatensatz entsprechend einem zweiten Röntgenstrahlungsenergiespektrums, das von dem ersten Röntgenstrahlungsenergiespektrum verschieden ist, erhalten. Durch Anwendung eines Dekompositionsmoduls, welches eine trainierte erste Funktion enthält, auf Eingangsdaten, welche von dem ersten Röntgenbilddatensatz und dem zweiten Röntgenbilddatensatz abhängen, wird wenigstens ein materialspezifischer Bilddatensatz erzeugt.

Sofern nicht anders angegeben, können alle Schritte des computerimplementierten Verfahrens von einer Datenverarbeitungsvorrichtung durchgeführt werden, welche wenigstens eine Recheneinheit aufweist. Insbesondere ist die wenigstens eine Recheneinheit zur Durchführung der Schritte des computerimplementierten Verfahrens konfiguriert oder angepasst. Hierzu kann die wenigstens eine Recheneinheit beispielsweise ein Computerprogramm speichern, das Befehle beinhaltet, die bei Ausführung durch die wenigstens eine Recheneinheit die wenigstens eine Recheneinheit dazu veranlassen, das computerimplementierte Verfahren auszuführen.

Aus jeder Ausführungsform des computerimplementierten Verfahrens kann jedoch direkt eine Ausführungsform eines Verfahrens abgeleitet werden, die nicht rein computerimplementiert ist, indem entsprechende Schritte zur Erzeugung des ersten Röntgenbilddatensatzes und des zweiten Röntgenbilddatensatzes, insbesondere mittels einer Röntgenquelle und einem Röntgendetektor, mit einbezogen werden.

Der erste Röntgenbilddatensatz und der zweite Röntgenbilddatensatz stellen jeweils denselben Bereich eines abzubildenden Objekts aus derselben Perspektive dar. Im Falle eines C-Bogen-Röntgengeräts oder einer Vorrichtung zur CT oder CBCT ist die Scanposition der Röntgenquelle und des Röntgendetektors bezüglich einander und bezüglich des Objekts bei der Erzeugung des ersten Röntgenbilddatensatzes und des zweiten Röntgenbilddatensatzes also insbesondere dieselbe.

Dass die Eingangsdaten von dem ersten Röntgenbilddatensatz und dem zweiten Röntgenbilddatensatz abhängen, kann insbesondere derart verstanden werden, dass die Eingangsdaten den ersten Röntgenbilddatensatz und den zweiten Röntgenbilddatensatz beinhalten oder jeweilige vorverarbeitete Varianten des ersten Röntgenbilddatensatzes und des zweiten Röntgenbilddatensatzes beinhalten. Die Vorverarbeitung kann beispielsweise eine Rauschreduzierung, eine sonstige Artefaktreduzierung, eine Bildfilterung und so weiter beinhalten.

Ein materialspezifischer Bilddatensatz kann im Kontext der Dual-Energie-Röntgenbildgebung beispielsweise derart verstanden werden, dass in dem materialspezifischen Bilddatensatz, insbesondere im Vergleich zum ersten Röntgenbilddatensatz und zweiten Röntgenbilddatensatz, ein bestimmtes Material besonders hervorgehoben oder unterdrückt ist. Um welches Material es sich dabei handelt hängt von der trainierten ersten Funktion ab, insbesondere von den zum Trainieren der ersten Funktion verwendeten Trainingsdaten. Der materialspezifische Bilddatensatz kann also beispielsweise Fett, Wasser, Blut, CSF, Kontrastmittel und so weiter hervorheben oder unterdrücken. Es kann sich auch um ein VNC-Bild handeln.

Die erste Funktion kann beispielsweise eine unter Verwendung von maschinellem Lernen trainierte Funktion sein. Die erste Funktion kann beispielsweise ein künstliches neuronales Netzwerk (KNN) sein, insbesondere ein faltendes neuronales Netzwerk, (CNN, englisch: convolutional neural network), oder ein Vision-Transformer-Netzwerk, oder eine Kombination mehrerer KNNs.

Die erste Funktion kann auch eine optimierbare beziehungsweise optimierte Funktion, beispielsweise eine mittels eines gradienten-basierten Verfahren optimierbare beziehungsweise optimierte Funktion, beinhalten. Beispielsweise kann ein bilateraler Filter nach den entsprechenden Parametern abgeleitet und damit optimiert werden.

Das Dekompositionsmodul kann auch Teil eines komplexeren Algorithmus sein, der neben der eigentlichen Materialdekomposition auch andere Aufgaben, wie etwa Vorverarbeitung, Filterung und/oder dreidimensionale Rekonstruktion aus mehreren Röntgenprojektionsbildern und so weiter beinhalten kann. Der erste Röntgenbilddatensatz und der zweite Röntgenbilddatensatz können insbesondere Röntgenprojektionsbilder oder dreidimensionale Rekonstruktionen basierend auf jeweils mehreren Röntgenprojektionsbildern sein.

Das erste Röntgenstrahlungsenergiespektrum und das zweite Röntgenstrahlungsenergiespektrum unterscheiden sich insbesondere in ihrer Strahlungsenergie, also Photonenenergie, oder spektralen Strahlungsenergieverteilung. Eine mittlere Strahlungsenergie des ersten Röntgenstrahlungsenergiespektrums ist insbesondere höher als eine mittlere Strahlungsenergie des zweiten Röntgenstrahlungsenergiespektrums. Die unterschiedlichen Röntgenstrahlungsenergiespektren können insbesondere durch unterschiedliche Spannungen, insbesondere Spitzenspannungen, mit denen die Röntgenquelle zur Erzeugung der jeweiligen Röntgenstrahlung betrieben wird, erreicht werden. Optional können zusätzlich auch Filter im Strahlengang, etwa zur Strahlaufhärtung bei dem Röntgenstrahlungsenergiespektrum mit der höheren mittleren Strahlungsenergie, verwendet werden.

Durch das erfindungsgemäße Verfahren kann eine hochqualitative automatische Materialdekomposition bei der Dual-Energie-Röntgenbildgebung erreicht werden, sodass insbesondere auch bei nicht optimaler Bildqualität der Röntgenbilddatensätze, etwa bei der CBCT, eine quantitative Analyse des materialspezifischen Bilddatensatzes ermöglicht wird.

Gemäß zumindest einer Ausführungsform entspricht der erste Röntgenbilddatensatz einem ersten Röntgenprojektionsbild und der zweite Röntgenbilddatensatz entspricht einem zweiten Röntgenprojektionsbild.

Gemäß zumindest einer Ausführungsform entspricht der erste Röntgenbilddatensatz einem ersten rekonstruierten Volumen und der zweite Röntgenbilddatensatz entspricht einem zweiten rekonstruierten Volumen.

Ein rekonstruiertes Volumen kann dabei als dreidimensionale Rekonstruktion des gesamten Objekts oder eines Teilbereichs des Objekts, insbesondere einer dreidimensionalen Schicht des Objekts, basierend auf Röntgenprojektionsbildern, die aus unterschiedlichen Richtungen aufgenommen wurden, beispielsweise in einem CT-Verfahren oder CBCT-Verfahren oder Thomosyntheseverfahren.

Gemäß zumindest einer Ausführungsform beinhaltet der wenigstens eine materialspezifische Bilddatensatz einen Kontrastmittelbilddatensatz.

Mit anderen Worten wurden der erste Röntgenbilddatensatz und der zweite Röntgenbilddatensatz nach einer Kontrastmittelgabe erzeugt, sodass sich also in dem Objekt das Kontrastmittel befand. Das Kontrastmittel kann ein jodhaltiges Kontrastmittel sein. Der Kontrastmittelbilddatensatz wird dann mitunter auch Jodbild genannt. Das Kontrastmittel kann auch ein bariumhaltiges Kontrastmittel oder ein sonstiges röntgenpositives oder röntgennegatives Kontrastmittel sein. Dies kann insbesondere vorteilhaft sein, wenn die Anreicherung des Kontrastmittels in einem Bereich des Objekts von besonderem Interesse ist.

Gemäß zumindest einer Ausführungsform beinhaltet der wenigstens eine materialspezifische Bilddatensatz einen Nicht-Kontrast-Bilddatensatz, insbesondere einen VNC-Bilddatensatz, also beispielsweise ein VNC-Bild oder eine dreidimensionale VNC-Rekonstruktion.

Mit anderen Worten wurden auch hier der erste Röntgenbilddatensatz und der zweite Röntgenbilddatensatz nach einer Kontrastmittelgabe erzeugt. Im Nicht-Kontrast-Bilddatensatz ist das Kontrastmittel unterdrückt, es stellt also eine Aufnahme nach, wie sie ohne Kontrastmittelgabe erhalten würde. Dies kann insbesondere vorteilhaft sein, wenn das Kontrastmittel als eigentlich nicht oder nicht mehr erwünschter Rückstand vorhanden ist.

Gemäß zumindest einer Ausführungsform werden durch Anwendung eines Artefaktreduktionsmoduls, welches wenigstens eine trainierte zweite Funktion enthält, auf weitere Eingangsdaten, welche von dem ersten Röntgenbilddatensatz und dem zweiten Röntgenbilddatensatz abhängen, diese beispielsweise beinhalten, ein artefaktreduzierter erster Röntgenbilddatensatz und artefaktreduzierter zweiter Röntgenbilddatensatz erzeugt. Die Eingangsdaten hängen von dem artefaktreduzierten ersten Röntgenbilddatensatz und dem artefaktreduzierten zweiten Röntgenbilddatensatz, beinhalten diese beispielsweise.

Die wenigstens eine zweite Funktion kann beispielsweise wenigstens eine unter Verwendung von maschinellem Lernen trainierte Funktion sein. Die wenigstens eine zweite Funktion kann beispielsweise ein KNN beinhalten, insbesondere CNN oder ein Vision-Transformer-Netzwerk, oder eine Kombination mehrerer KNNs.

Je nach Ausgestaltungsform kann eine trainierte zweite Funktion, also beispielsweise ein trainiertes KNN, der wenigstens einen trainierten zweiten Funktion auf den ersten Röntgenbilddatensatz und den zweiten Röntgenbilddatensatz angewendet werden, um den artefaktreduzierten ersten Röntgenbilddatensatz und den artefaktreduzierten zweiten Röntgenbilddatensatz zu erzeugen.

In anderen Ausführungsformen kann eine trainierte zweite Funktion der wenigstens einen trainierten zweiten Funktion auf den ersten Röntgenbilddatensatz angewendet werden, um den artefaktreduzierten ersten Röntgenbilddatensatz zu erzeugen, und eine trainierte weitere zweite Funktion der wenigstens einen trainierten zweiten Funktion auf den zweiten Röntgenbilddatensatz, um den artefaktreduzierten zweiten Röntgenbilddatensatz zu erzeugen.

Die wenigstens eine trainierte zweite Funktion ist insbesondere dazu trainiert, in Röntgenbilddatensätzen Artefakte zu reduzieren. Die Artefakte können Rauschen beinhalten oder sonstige Artefakte, beispielsweise aufgrund von Streustrahlung oder Trunkierung, Kegelstrahlartefakte, und so weiter beinhalten.

Dadurch wird die Qualität der Eingangsdaten für das Dekompositionsmodul erhöht und dementsprechend auch die Qualität des wenigstens einen materialspezifischen Bilddatensatzes.

Eine zweite Funktion kann auch eine optimierbare beziehungsweise optimierte Funktion, beispielsweise eine mittels eines gradienten-basierten Verfahren optimierbare beziehungsweise optimierte Funktion, beinhalten. Beispielsweise kann ein bilateraler Filter nach den entsprechenden Parametern abgeleitet und damit optimiert werden.

Die erste Funktion und die wenigstens eine zweite Funktion können gemeinsam oder separat voneinander trainiert werden.

Gemäß zumindest einer Ausführungsform werden durch Anwendung eines Filtermoduls, welches wenigstens eine Filterfunktion enthält, auf weitere Eingangsdaten, welche von dem ersten Röntgenbilddatensatz und dem zweiten Röntgenbilddatensatz abhängen, diese beispielsweise enthalten, ein gefilterter erster Röntgenbilddatensatz und ein gefilterter zweiter Röntgenbilddatensatz erzeugt. Die Eingangsdaten hängen von dem gefilterten ersten Röntgenbilddatensatz und dem gefilterten zweiten Röntgenbilddatensatz ab, enthalten diese beispielsweise.

Die wenigstens eine Filterfunktion kann beispielsweise eine unter Verwendung von maschinellem Lernen trainierte Funktion sein. Die wenigstens eine zweite Funktion kann beispielsweise ein KNN beinhalten, insbesondere CNN oder ein Vision-Transformer-Netzwerk, oder eine Kombination mehrerer KNNs. Die wenigstens eine Filterfunktion kann aber auch eine wenigstens eine konventionelle Filterfunktion sein, die also nicht unter Verwendung von maschinellem Lernen trainiert wurde. In diesem Fall können Parameter der wenigstens eine Filterfunktion vorgegeben sein und/oder je nach Anwendungsfall manuell oder automatisch variiert werden.

Die wenigstens eine Filterfunktion kann beispielsweise zur Kontrastverbesserung, zur Rauschreduzierung, zur Kantenverbesserung, und so weiter dienen.

Je nach Ausgestaltungsform kann eine Filterfunktion der wenigstens einen Filterfunktion auf den ersten Röntgenbilddatensatz und den zweiten Röntgenbilddatensatz angewendet werden, um den gefilterten ersten Röntgenbilddatensatz und den gefilterten zweiten Röntgenbilddatensatz zu erzeugen.

In anderen Ausführungsformen kann eine Filterfunktion der wenigstens einen Filterfunktion auf den ersten Röntgenbilddatensatz angewendet werden, um den gefilterten ersten Röntgenbilddatensatz zu erzeugen, und eine weitere Filterfunktion der wenigstens einen Filterfunktion auf den zweiten Röntgenbilddatensatz, um den gefilterten zweiten Röntgenbilddatensatz zu erzeugen.

Dadurch wird die Qualität der Eingangsdaten für das Dekompositionsmodul erhöht und dementsprechend auch die Qualität des wenigstens einen materialspezifischen Bilddatensatzes.

Ausführungsformen in denen die Anwendung des Filtermoduls vorgesehen ist können auch mit Ausführungsformen in denen die Anwendung des Artefaktreduktionsmoduls vorgesehen ist kombiniert werden. In diesem Fall realisieren das Artefaktreduktionsmodul und das Filtermodul insbesondere unterschiedliche Rauschreduzierungsalgorithmen oder dienen nicht beide zur Rauschreduzierung. Dabei kann beispielsweise das Artefaktreduktionsmodul auf erste weitere Eingangsdaten, welche von dem ersten Röntgenbilddatensatz und dem zweiten Röntgenbilddatensatz abhängen, diese insbesondere enthalten, angewendet werden, um den artefaktreduzierten ersten Röntgenbilddatensatz und den artefaktreduzierten zweiten Röntgenbilddatensatz zu erzeugt. Das Filtermodul kann dann auf zweite weitere Eingangsdaten, welche von dem artefaktreduzierten ersten Röntgenbilddatensatz und dem artefaktreduzierten zweiten Röntgenbilddatensatz abhängen, diese insbesondere enthalten, angewendet werden, um den gefilterten ersten Röntgenbilddatensatz und den gefilterten zweiten Röntgenbilddatensatz zu erzeugen. Es kann auch umgekehrt vorgegangen werden, also zuerst das Filtermodul und danach das Artefaktreduktionsmodul angewendet werden.

Gemäß zumindest einer Ausführungsform enthält die wenigstens eine Filterfunktion wenigstens eine Filterfunktion zur bilateralen Filterung und/oder wenigstens eine Filterfunktion zur differenzierbaren geführten Filterung (englisch: differentiable guided filter).

Gemäß einem weiteren Aspekt der Erfindung wird ein Verfahren zur Dual-Energie-Röntgenbildgebung angegeben. Dabei wird ein erster Röntgenbilddatensatz, welcher ein abzubildendes Objekt darstellt, erzeugt, indem erste Röntgenstrahlung entsprechend einem ersten Röntgenstrahlungsenergiespektrum erzeugt wird, insbesondere mittels einer Röntgenquelle, und durch das Objekt hindurchtretende Anteile der ersten Röntgenstrahlung detektiert werden, insbesondere mittels eines Röntgendetektors. Ein zweiter Röntgenbilddatensatz, welcher das Objekt darstellt, wird erzeugt, indem zweite Röntgenstrahlung entsprechend einem zweiten Röntgenstrahlungsenergiespektrum, insbesondere mittels der Röntgenquelle, erzeugt wird und durch das Objekt hindurchtretende Anteile der ersten Röntgenstrahlung detektiert werden, insbesondere mittels des Röntgendetektors. Ein erfindungsgemäßes computerimplementiertes Verfahren zur Materialdekomposition wird unter Verwendung des ersten Röntgenbilddatensatzes und des zweiten Röntgenbilddatensatzes durchgeführt.

Gemäß zumindest einer Ausführungsform des Verfahrens zur Dual-Energie-Röntgenbildgebung wird das Verfahren als CT-Verfahren durchgeführt.

Gemäß zumindest einer Ausführungsform des Verfahrens zur Dual-Energie-Röntgenbildgebung wird das Verfahren als CBCT-Verfahren durchgeführt.

Hier wirkt sich die Erfindung aufgrund der tendenziell geringeren Bildqualität bei CBCT-Verfahren besonders vorteilhaft aus.

Gemäß zumindest einer Ausführungsform entspricht der erste Röntgenbilddatensatz einem ersten Röntgenprojektionsbild, und der zweiten Röntgenbilddatensatz entspricht einem zweiten Röntgenprojektionsbild. Wenigstens ein rekonstruiertes Volumen wird abhängig von dem wenigstens einen materialspezifischen Bilddatensatz erzeugt.

Zur Erzeugung des rekonstruierten Volumens abhängig von dem wenigstens einen materialspezifischen Bilddatensatz können bekannte Rekonstruktionsverfahren genutzt werden. Insbesondere wird eine Vielzahl materialspezifischer Bilddatensätze aus unterschiedlichen Blickrichtungen erzeugt und das rekonstruierte Volumen wird abhängig von der Vielzahl materialspezifischer Bilddatensätze erzeugt.

Gemäß zumindest einer Ausführungsform entspricht der erste Röntgenbilddatensatz einem ersten rekonstruierten Volumen und der zweite Röntgenbilddatensatz entspricht einem zweiten rekonstruierten Volumen.

Das erste rekonstruierten Volumen und das zweite rekonstruierte Volumen können unter Verwendung bekannter Rekonstruktionsverfahren erzeugt werden. Insbesondere wird für das erste und das zweite Röntgenstrahlungsenergiespektrum jeweils eine Vielzahl von Röntgenprojektionsbildern aus unterschiedlichen Blickrichtungen erzeugt und das jeweilige rekonstruierte Volumen wird abhängig von der jeweiligen Vielzahl von Röntgenprojektionsbildern erzeugt.

Gemäß einem weiteren Aspekt der Erfindung wird ein computerimplementiertes Trainingsverfahren zur Bereitstellung einer trainierten ersten Funktion zur Verwendung in einem erfindungsgemäßen computerimplementierten Verfahren zur Materialdekomposition bei der Dual-Energie-Röntgenbildgebung angegeben. Ein erster Röntgentrainingsbilddatensatz entsprechend einem ersten Röntgenstrahlungsenergiespektrum und ein zweiter Röntgentrainingsbilddatensatz entsprechend einem zweiten Röntgenstrahlungsenergiespektrum werden erhalten. Wenigstens ein materialspezifischer Ground-Truth-Bilddatensatz für den ersten Röntgentrainingsbilddatensatz und den zweiten Röntgentrainingsbilddatensatz wird erhalten. Durch Anwendung eines Dekompositionsmoduls, welches die untrainierte oder teilweise trainierte erste Funktion enthält, auf Eingangstrainingsdaten, welche von dem ersten Röntgentrainingsbilddatensatz und dem zweiten Röntgentrainingsbilddatensatz abhängen, wird wenigstens ein prädizierter materialspezifischer Bilddatensatz erzeugt, insbesondere prädiziert.

Eine vorgegebene Verlustfunktion, die von dem wenigstens einen prädizierten materialspezifischen Bilddatensatz und dem wenigstens einen materialspezifischen Ground-Truth-Bilddatensatz abhängt, insbesondere von einer Abweichung zwischen dem wenigstens einen prädizierten materialspezifischen Bilddatensatz und dem wenigstens einen materialspezifischen Ground-Truth-Bilddatensatz, wird ausgewertet. Parameter der ersten Funktion werden abhängig von einem Ergebnis der Auswertung der Verlustfunktion, insbesondere von einem Wert der Verlustfunktion, aktualisiert.

Die erste Funktion ist insbesondere eine unter Verwendung von maschinellem Lernen trainierbare Funktion, beispielsweise ein KNN oder eine Kombination mehrerer KNNs. Die Parameter der ersten Funktion enthalten dann beispielsweise Gewichtungsfaktoren und/oder Bias-Faktoren des KNN oder der KNNs.

Die Schritte des Trainingsverfahrens können insbesondere für eine Vielzahl von ersten und zweiten Röntgentrainingsbilddatensätzen und zugehörigen Ground-Truth-Bilddatensätzen wiederholt werden, bis ein vorgegebenes Abbruchkriterium oder Konvergenzkriterium für die Verlustfunktion erfüllt ist.

Enthält der wenigstens eine prädizierte materialspezifische Bilddatensatz mehrere prädizierte materialspezifische Bilddatensatz, so kann der wenigstens eine materialspezifische Ground-Truth-Bilddatensatz einen materialspezifischen Ground-Truth-Bilddatensatz für jeden prädizierten materialspezifischen Bilddatensatz beinhalten. Die Verlustfunktion kann dann beispielsweise einen jeweiligen Verlustterm für jedes Paar eines prädizierten materialspezifischen Bilddatensatzes und des zugehörigen materialspezifischen Ground-Truth-Bilddatensatzes beinhalten.

Gemäß zumindest einer Ausführungsform des computerimplementierten Trainingsverfahrens ist dieses als Trainingsverfahren zur Bereitstellung der trainierten ersten Funktion und wenigstens einer trainierten zweiten Funktion ausgestaltet. Durch Anwendung eines Artefaktreduktionsmoduls, welches die wenigstens eine untrainierte oder teilweise trainierte zweite Funktion enthält, auf weitere Eingangstrainingsdaten, welche von dem ersten Röntgentrainingsbilddatensatz und dem zweiten Röntgentrainingsbilddatensatz abhängen, diese insbesondere enthalten, werden ein prädizierter artefaktreduzierter erster Röntgenbilddatensatz und ein prädizierter artefaktreduzierter zweiter Röntgenbilddatensatz erzeugt. Die Eingangstrainingsdaten hängen von dem prädizierten artefaktreduzierten ersten Röntgenbilddatensatz und dem prädizierten artefaktreduzierten zweiten Röntgenbilddatensatz ab, enthalten diese insbesondere.

Gemäß zumindest einer Ausführungsform werden Parameter der wenigstens einen zweiten Funktion abhängig von einem Ergebnis der Auswertung der Verlustfunktion aktualisiert.

In solchen Ausführungsformen werden die erste Funktion und die wenigstens eine zweite Funktion Ende-zu-Ende trainiert, was eine geringere Menge an Trainingsdaten erfordert und dazu führt, dass die beiden Aufgaben der Artefaktreduzierung und der Materialdekomposition optimal aufeinander abgestimmt trainiert werden.

Die wenigstens eine zweite Funktion ist insbesondere wenigstens eine unter Verwendung von maschinellem Lernen trainierbare Funktion, beispielsweise ein KNN oder eine Kombination mehrerer KNNs. Die Parameter der wenigstens einen zweiten Funktion enthalten dann beispielsweise Gewichtungsfaktoren und/oder Bias-Faktoren des KNN oder der KNNs.

Gemäß zumindest einer Ausführungsform werden ein artefaktreduzierter erster Ground-Truth-Röntgenbilddatensatz für den ersten Röntgentrainingsbilddatensatz und ein artefaktreduzierter zweiter Ground-Truth-Röntgenbilddatensatz für den zweiten Röntgentrainingsbilddatensatz erhalten. Eine vorgegebene weitere Verlustfunktion, die einen Verlustterm enthält, welcher von dem prädizierten artefaktreduzierten ersten Röntgenbilddatensatz und dem artefaktreduzierten ersten Ground-Truth-Röntgenbilddatensatz abhängt, insbesondere von einer entsprechenden Abweichung, sowie einen weiteren Verlustterm, welcher von dem prädizierten artefaktreduzierten zweiten Röntgenbilddatensatz und dem artefaktreduzierten zweiten Ground-Truth-Röntgenbilddatensatz abhängt, insbesondere von einer entsprechenden Abweichung, wird ausgewertet. Die Parameter der wenigstens einen zweiten Funktion werden abhängig von einem Ergebnis der Auswertung der weiteren Verlustfunktion aktualisiert.

Die wenigstens eine zweite Funktion wird also unabhängig von der ersten Funktion trainiert. Dies kann vorteilhaft sein, wenn die wenigstens eine zweite Funktion beispielsweise für unterschiedliche Anwendungen bereitgestellt werden soll.

Gemäß zumindest einer Ausführungsform des computerimplementierten Trainingsverfahrens ist dieses als Trainingsverfahren zur Bereitstellung der trainierten ersten Funktion und wenigstens einer Filterfunktion ausgestaltet. Durch Anwendung eines Filtermoduls, welches die wenigstens eine Filterfunktion enthält, auf weitere Eingangstrainingsdaten, welche von dem ersten Röntgentrainingsbilddatensatz und dem zweiten Röntgentrainingsbilddatensatz abhängen, diese insbesondere enthalten, werden ein prädizierter gefilterter erster Röntgenbilddatensatz und ein prädizierter gefilterter zweiter Röntgenbilddatensatz erzeugt. Die Eingangstrainingsdaten hängen von dem prädizierten gefilterten ersten Röntgenbilddatensatz und dem prädizierten gefilterten zweiten Röntgenbilddatensatz ab.

Gemäß zumindest einer Ausführungsform wird wenigstens ein Parameter der wenigstens einen Filterfunktion abhängig von einem Ergebnis der Auswertung der Verlustfunktion aktualisiert. In solchen Ausführungsformen werden die erste Funktion und die wenigstens eine Filterfunktion Ende-zu-Ende trainiert, was eine geringere Menge an Trainingsdaten erfordert und dazu führt, dass die beiden Aufgaben der Filterung und der Materialdekomposition optimal aufeinander abgestimmt trainiert werden.

Die wenigstens eine Filterfunktion kann insbesondere wenigstens eine unter Verwendung von maschinellem Lernen trainierbare Funktion, beispielsweise ein KNN oder eine Kombination mehrerer KNNs, sein. Die Parameter der wenigstens einen Filterfunktion enthalten dann beispielsweise Gewichtungsfaktoren und/oder Bias-Faktoren des KNN oder der KNNs.

Die wenigstens eine Filterfunktion kann aber auch einen konventionellen Filteralgorithmus, beispielsweise einen bilateralen Filter, beinhalten. Die Parameter der wenigstens einen Filterfunktion enthalten dann beispielsweise eine Kernelgröße eines ersten Filterkernels und/oder eine Kernelgröße eines zweiten Filterkernels, beispielsweise eine Standardabweichung im Falle eines Gauß'schen Filterkernels, und/oder eine Fenstergröße des Filters beinhalten.

Gemäß zumindest einer Ausführungsform werden ein gefilterter erster Ground-Truth-Röntgenbilddatensatz für den ersten Röntgentrainingsbilddatensatz und ein gefilterter zweiter Ground-Truth-Röntgenbilddatensatz für den zweiten Röntgentrainingsbilddatensatz erhalten. Eine vorgegebene weitere Verlustfunktion wird ausgewertet, die einen Verlustterm, welcher von dem prädizierten gefilterten ersten Röntgenbilddatensatz und dem gefilterten ersten Ground-Truth-Röntgenbilddatensatz abhängt, insbesondere von einer entsprechenden Abweichung, sowie einen weiteren Verlustterm, welcher von dem prädizierten gefilterten zweiten Röntgenbilddatensatz und dem gefilterten zweiten Ground-Truth-Röntgenbilddatensatz abhängt, insbesondere von einer entsprechenden Abweichung, enthält. Die Parameter der wenigstens einen Filterfunktion werden abhängig von einem Ergebnis der Auswertung der weiteren Verlustfunktion aktualisiert.

Die wenigstens eine Filterfunktion wird also unabhängig von der ersten Funktion trainiert. Dies kann vorteilhaft sein, wenn die wenigstens eine Filterfunktion beispielsweise für unterschiedliche Anwendungen bereitgestellt werden soll.

Gemäß einem weiteren Aspekt der Erfindung wird eine Datenverarbeitungsvorrichtung angegeben. Die Datenverarbeitungsvorrichtung weist wenigstens eine Recheneinheit auf, die dazu angepasst ist, ein erfindungsgemäßes computerimplementiertes Verfahren zur Materialdekomposition bei der Dual-Energie-Röntgenbildgebung und/oder ein erfindungsgemäßes computerimplementiertes Trainingsverfahren durchzuführen.

Unter einer Recheneinheit kann insbesondere ein Datenverarbeitungsgerät verstanden werden, das einen Verarbeitungsschaltkreis enthält. Die Recheneinheit kann also insbesondere Daten zur Durchführung von Rechenoperationen verarbeiten. Darunter fallen gegebenenfalls auch Operationen, um indizierte Zugriffe auf eine Datenstruktur, beispielsweise eine Umsetzungstabelle, LUT (englisch: "look-up table"), durchzuführen.

Die Recheneinheit kann insbesondere einen oder mehrere Computer, einen oder mehrere Mikrocontroller und/oder einen oder mehrere integrierte Schaltkreise enthalten, beispielsweise eine oder mehrere anwendungsspezifische integrierte Schaltungen, ASIC (englisch: "application specific integrated circuit"), eines oder mehrere feldprogrammierbare Gate Arrays, FPGA, und/oder eines oder mehrere Einchipsysteme, SoC (englisch: "system on a chip"). Die Recheneinheit kann auch einen oder mehrere Prozessoren, beispielsweise einen oder mehrere Mikroprozessoren, eine oder mehrere zentrale Prozessoreinheiten, CPU (englisch: "central processing unit"), eine oder mehrere Grafikprozessoreinheiten, GPU (englisch: "graphics processing unit") und/oder einen oder mehrere Signalprozessoren, insbesondere einen oder mehrere Digitalsignalprozessoren, DSP, enthalten. Die Recheneinheit kann auch einen physischen oder einen virtuellen Verbund von Computern oder sonstigen der genannten Einheiten beinhalten.

In verschiedenen Ausführungsbeispielen beinhaltet die Recheneinheit eine oder mehrere Hardware- und/oder Softwareschnittstellen und/oder eine oder mehrere Speichereinheiten.

Eine Speichereinheit kann als flüchtiger Datenspeicher, beispielsweise als dynamischer Speicher mit wahlfreiem Zugriff, DRAM (englisch: "dynamic random access memory") oder statischer Speicher mit wahlfreiem Zugriff, SRAM (englisch: "static random access memory"), oder als nicht-flüchtiger Datenspeicher, beispielsweise als Festwertspeicher, ROM (englisch: "read-only memory"), als programmierbarer Festwertspeicher, PROM (englisch: "programmable read-only memory"), als löschbarer programmierbarer Festwertspeicher, EPROM (englisch: "erasable programmable read-only memory"), als elektrisch löschbarer programmierbarer Festwertspeicher, EEPROM (englisch: "electrically erasable programmable read-only memory"), als Flash-Speicher oder Flash-EEPROM, als ferroelektrischer Speicher mit wahlfreiem Zugriff, FRAM (englisch: "ferroelectric random access memory"), als magnetoresistiver Speicher mit wahlfreiem Zugriff, MRAM (englisch: "magnetoresistive random access memory") oder als Phasenänderungsspeicher mit wahlfreiem Zugriff, PCRAM (englisch: "phase-change random access memory"), ausgestaltet sein.

Gemäß einem weiteren Aspekt der Erfindung wird eine Röntgenbildgebungsvorrichtung aufweisend eine erfindungsgemäße Datenverarbeitungsvorrichtung, eine Röntgenquelle, einen Röntgendetektor und wenigstens eine Steuereinheit angegeben. Die wenigstens eine Steuereinheit ist dazu eingerichtet, die Röntgenquelle anzusteuern, erste Röntgenstrahlung entsprechend einem ersten Röntgenstrahlungsenergiespektrum zu erzeugen. Der Röntgendetektor ist dazu eingerichtet, den ersten Röntgenbilddatensatz, welcher ein abzubildendes Objekt darstellt, zu erzeugen und dazu durch das Objekt hindurchtretende Anteile der ersten Röntgenstrahlung zu detektieren. Die wenigstens eine Steuereinheit ist dazu eingerichtet, die Röntgenquelle anzusteuern, zweite Röntgenstrahlung entsprechend einem zweiten Röntgenstrahlungsenergiespektrum zu erzeugen. Der Röntgendetektor ist dazu eingerichtet, den zweiten Röntgenbilddatensatz, welcher das Objekt darstellt, zu erzeugen und dazu durch das Objekt hindurchtretende Anteile der zweiten Röntgenstrahlung zu detektieren.

Dass der Röntgendetektor den ersten und den zweiten Röntgenbilddatensatz erzeugt, kann insbesondere derart verstanden werden, dass der Röntgendetektor das erste Röntgenprojektionsbild als ersten Röntgenbilddatensatz erzeugt und das zweite Röntgenprojektionsbild als zweiten Röntgenbilddatensatz. Falls die Röntgenbilddatensätze rekonstruierte Volumina sind, erzeugt der Röntgendetektor die entsprechenden Röntgenprojektionsbilder und die wenigstens eine Recheneinheit erzeugt die Röntgenbilddatensätze.

Die wenigstens eine Steuereinheit kann beispielsweise Teil der wenigstens einen Recheneinheit der Datenverarbeitungsvorrichtung sein.

Weitere Ausführungsformen der erfindungsgemäßen Röntgenbildgebungsvorrichtung folgen unmittelbar aus den verschiedenen Ausgestaltungen des erfindungsgemäßen computerimplementierten Verfahrens und des erfindungsgemäßen computerimplementierten Trainingsverfahrens und jeweils umgekehrt. Insbesondere lassen sich einzelne Merkmale und entsprechende Erläuterungen sowie Vorteile bezüglich der verschiedenen Ausführungsformen zu dem erfindungsgemäßen Verfahren und dem erfindungsgemäßen Trainingsverfahren analog auf entsprechende Ausführungsformen der erfindungsgemäßen Röntgenbildgebungsvorrichtung übertragen. Insbesondere ist die erfindungsgemäße Röntgenbildgebungsvorrichtung zum Durchführen eines erfindungsgemäßen computerimplementierten Verfahrens ausgebildet oder programmiert. Insbesondere führt die erfindungsgemäße Röntgenbildgebungsvorrichtung das erfindungsgemäße computerimplementierte Verfahren durch.

Gemäß einem weiteren Aspekt der Erfindung wird ein Computerprogramm mit Befehlen angegeben. Wenn die Befehle durch eine Datenverarbeitungsvorrichtung ausgeführt werden, veranlassen die Befehle die Datenverarbeitungsvorrichtung dazu, ein erfindungsgemäßes computerimplementiertes Verfahren zur Materialdekomposition und/oder ein erfindungsgemäßes computerimplementiertes Trainingsverfahren durchzuführen.

Die Befehle können beispielsweise als Programmcode vorliegen. Der Programmcode kann beispielsweise als Binärcode oder Assembler und/oder als Quellcode einer Programmiersprache, zum Beispiel C, und/oder als Programmskript, zum Beispiel Python, bereitgestellt sein.

Gemäß einem weiteren Aspekt der Erfindung wird ein weiteres Computerprogramm mit weiteren Befehlen angegeben. Wenn die weiteren Befehle durch eine erfindungsgemäße Röntgenbildgebungsvorrichtung ausgeführt werden, insbesondere durch die Datenverarbeitungsvorrichtung der Röntgenbildgebungsvorrichtung, veranlassen die weiteren Befehle die Röntgenbildgebungsvorrichtung dazu, ein erfindungsgemäßes Verfahren zur Dual-Energie-Röntgenbildgebung durchzuführen.

Die weiteren Befehle können beispielsweise als Programmcode vorliegen. Der Programmcode kann beispielsweise als Binärcode oder Assembler und/oder als Quellcode einer Programmiersprache, zum Beispiel C, und/oder als Programmskript, zum Beispiel Python, bereitgestellt sein.

Gemäß einem weiteren Aspekt der Erfindung wird ein computerlesbares Speichermedium angegeben, das ein erfindungsgemäßes Computerprogramm und/oder ein erfindungsgemäßes weiteres Computerprogramm speichert.

Das Computerprogramm, das weitere Computerprogramm und das computerlesbare Speichermedium sind jeweils Computerprogrammprodukte mit den Befehlen und/oder den weiteren Befehlen.

Weitere Merkmale und Merkmalskombinationen der Erfindung ergeben sich aus den Figuren und deren Beschreibung sowie aus den Ansprüchen. Insbesondere müssen weitere Ausführungsformen der Erfindung nicht unbedingt alle Merkmale eines der Ansprüche enthalten. Weitere Ausführungsformen der Erfindungen können Merkmale oder Merkmalskombinationen aufweisen, die nicht in den Ansprüchen genannt sind.

Die Erfindung wird im Folgenden anhand konkreter Ausführungsbeispiele und zugehöriger schematischer Zeichnungen näher erläutert. In den Figuren können gleiche oder funktionsgleiche Elemente mit denselben Bezugszeichen versehen sein. Die Beschreibung gleicher oder funktionsgleicher Elemente wird gegebenenfalls nicht notwendigerweise bezüglich verschiedener Figuren wiederholt.

In den Figuren zeigen
- FIG 1: eine schematische Darstellung einer beispielhaften Ausführungsform einer erfindungsgemäßen Röntgenbildgebungsvorrichtung;
- FIG 2: eine schematische Prinzipdarstellung einer DreiMaterialdekomposition bei der Dual-EnergieRöntgenbildgebung;
- FIG 3: eine schematische Darstellung einer beispielhaften Ausführungsform eines erfindungsgemäßen computerimplementierten Verfahrens zur Materialdekomposition beziehungsweise eines entsprechenden computerimplementierten Trainingsverfahrens;
- FIG 4: eine schematische Darstellung einer weiteren beispielhaften Ausführungsform eines erfindungsgemäßen computerimplementierten Verfahrens zur Materialdekomposition beziehungsweise eines entsprechenden computerimplementierten Trainingsverfahrens;
- FIG 5: eine schematische Darstellung einer weiteren beispielhaften Ausführungsform eines erfindungsgemäßen computerimplementierten Verfahrens zur Materialdekomposition beziehungsweise eines entsprechenden computerimplementierten Trainingsverfahrens;
- FIG 6: eine schematische Darstellung einer weiteren beispielhaften Ausführungsform eines erfindungsgemäßen computerimplementierten Verfahrens zur Materialdekomposition beziehungsweise eines entsprechenden computerimplementierten Trainingsverfahrens;
- FIG 7: eine schematische Darstellung einer weiteren beispielhaften Ausführungsform eines erfindungsgemäßen computerimplementierten Verfahrens zur Materialdekomposition beziehungsweise eines entsprechenden computerimplementierten Trainingsverfahrens;
- FIG 8: eine schematische Darstellung einer weiteren beispielhaften Ausführungsform eines erfindungsgemäßen computerimplementierten Verfahrens zur Materialdekomposition beziehungsweise eines entsprechenden computerimplementierten Trainingsverfahrens;
- FIG 9: eine schematische Darstellung einer weiteren beispielhaften Ausführungsform eines erfindungsgemäßen computerimplementierten Verfahrens zur Materialdekomposition beziehungsweise eines entsprechenden computerimplementierten Trainingsverfahrens;
- FIG 10: ein schematisches Ablaufdiagramm einer beispielhaften Ausführungsform eines erfindungsgemäßen Verfahrens zur Dual-Energie-Röntgenbildgebung;
- FIG 11: ein schematisches Ablaufdiagramm einer weiteren beispielhaften Ausführungsform eines erfindungsgemäßen Verfahrens zur Dual-Energie-Röntgenbildgebung.

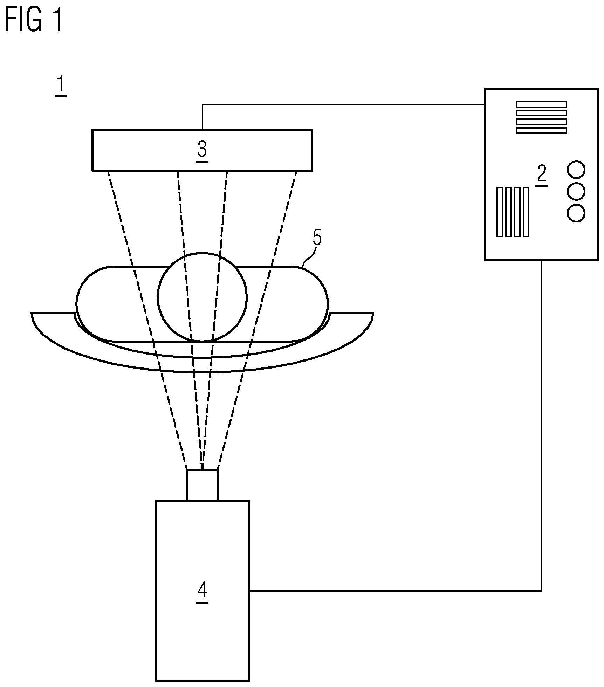

FIG 1 zeigt eine schematische Darstellung einer beispielhaften Ausführungsform einer erfindungsgemäßen Röntgenbildgebungsvorrichtung 1, beispielsweise zur CBCT.

Die Röntgenbildgebungsvorrichtung weist eine erfindungsgemäße Datenverarbeitungsvorrichtung 2 auf, eine Röntgenquelle 4, einen Röntgendetektor 3 und wenigstens eine Steuereinheit, die beispielsweise Teil der Datenverarbeitungsvorrichtung 2 sein kann. Die wenigstens eine Steuereinheit ist dazu eingerichtet, die Röntgenquelle 4 dazu anzusteuern, erste Röntgenstrahlung entsprechend einem ersten Röntgenstrahlungsenergiespektrum zu erzeugen. Der Röntgendetektor 3 ist dazu eingerichtet, erste Röntgenprojektionsbilder, welche ein abzubildendes Objekt 5 darstellen, zu erzeugen und dazu durch das Objekt 5 hindurchtretende Anteile der ersten Röntgenstrahlung zu detektieren. Die wenigstens eine Steuereinheit dazu eingerichtet ist, die Röntgenquelle 4 anzusteuern, zweite Röntgenstrahlung entsprechend einem zweiten Röntgenstrahlungsenergiespektrum zu erzeugen. Der Röntgendetektor ist dazu eingerichtet, zweite Röntgenprojektionsbilder, welche das Objekt 5 darstellen, zu erzeugen und dazu durch das Objekt 5 hindurchtretende Anteile der zweiten Röntgenstrahlung zu detektieren.

Die Datenverarbeitungsvorrichtung 2 weist wenigstens eine Recheneinheit auf, die dazu eingerichtet ist, ein erfindungsgemäßes computerimplementiertes Verfahren zur Materialdekomposition und ein entsprechendes erfindungsgemäßes computerimplementiertes Trainingsverfahren durchzuführen.

Dabei nutzt die Datenverarbeitungsvorrichtung 2 ein erstes Röntgenprojektionsbild als ersten Röntgenbilddatensatz und ein zweites Röntgenprojektionsbild als zweiten Röntgenbilddatensatz oder erzeugt basierend auf den ersten Röntgenprojektionsbildern eine erste Volumenrekonstruktion 11, 21 als ersten Röntgenbilddatensatz und basierend auf den zweiten Röntgenprojektionsbildern eine zweite Volumenrekonstruktion 12, 22 als zweiten Röntgenbilddatensatz

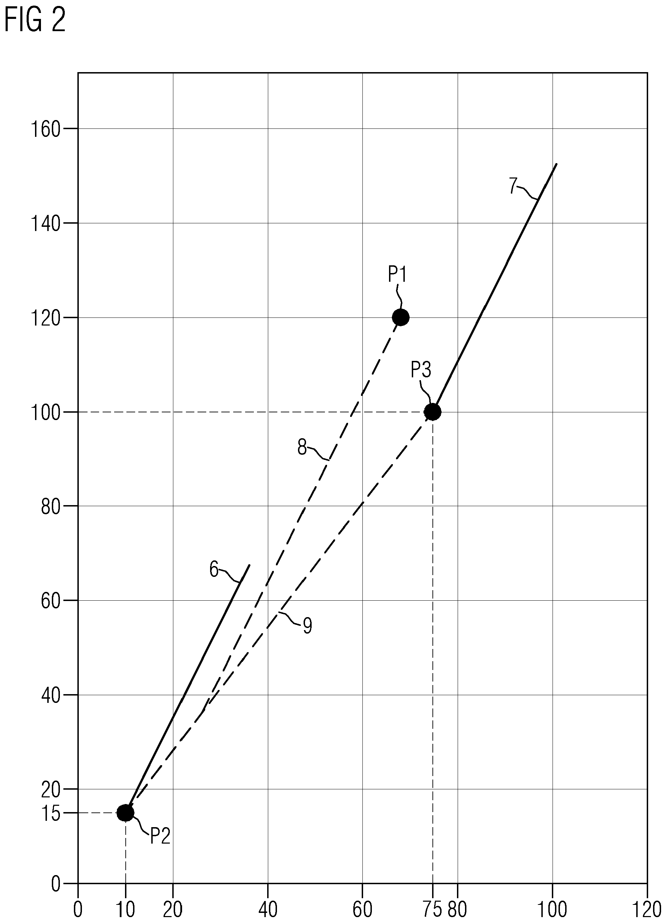

In FIG 2 ist eine schematische Prinzipdarstellung einer Drei-Material-Dekomposition bei der Dual-Energie-Röntgenbildgebung mit einem CT- oder CBCT-Verfahren gezeigt.

Auf der horizontalen Achse ist ein Abschwächungswert gemäß dem ersten Röntgenstrahlungsenergiespektrum aufgetragen, beispielsweise entsprechend einer Röhrenspannung der Röntgenquelle 4 von 125 kV, auf der vertikalen Achse ist der Abschwächungswert gemäß dem zweiten Röntgenstrahlungsenergiespektrum aufgetragen, beispielsweise entsprechend einer Röhrenspannung der Röntgenquelle 4 von 70 kV. Die Abschwächungswerte sind insbesondere in Hounsfield-Einheiten HE angegeben. Jedem Voxel einer dreidimensionalen Volumenrekonstruktion kann dann eine zweidimensionale Koordinate in der Darstellung der FIG 2 zugeordnet werden. In FIG 2 entspricht der Punkt P1 der gemessenen Abschwächung eines Voxels, der Punkt P2 der Abschwächung eines ersten Referenzmaterials, beispielsweise CSF, und der Punkt P3 der Abschwächung eines zweiten Referenzmaterials, beispielsweise einer Blutung. Eine Abschwächung von Jod als drittes Referenzmaterial ist in FIG 2 nicht dargestellt, da der entsprechende Punkt P4 bei sehr viel höheren Werten läge.

Die Linie 6 geht von Punkt P2 aus und ist parallel zur Verbindungslinie zwischen P2 und P4. Die Linie 7 geht von Punkt P3 aus und ist parallel zur Verbindungslinie zwischen P3 und P4. Die Linie 8 geht von Punkt P1 aus und ist parallel zur Verbindungslinie zwischen P1 und P4. Die Linie 9 verbindet die Punkte P2 und P3 miteinander. Die Linie 8 schneidet die Linie 9 und die Länge des Abschnitts der Linie 8 zwischen dem Punkt P1 und dem Schnittpunkt mit der Linie 9 kann als Näherungswert für die Abschwächung bei Punkt P1 aufgrund von Jod interpretiert werden. Sind die Punkte P1, P2, P3 und P4 gegeben, so kann also für jedes Voxel die jodbedingte Abschwächung berechnet werden und daraus ein entsprechende Kontrastmittelbild oder eine Kontrastmittelrekonstruktion erzeugt werden. Analog kann auch für die anderen Materialien vorgegangen werden. Das erfindungsgemäße computerimplementierte Verfahren zur Materialdekomposition kann beispielsweise dieselben Ergebnisse allein basierend auf einer trainierten ersten Funktion erzielen.

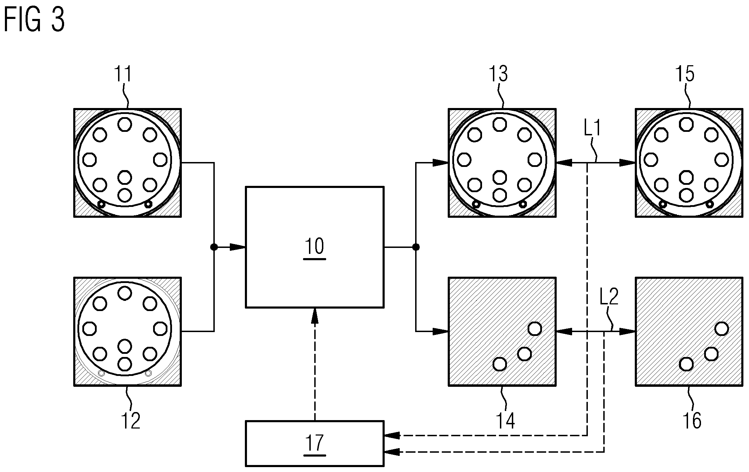

Eine schematische Darstellung einer beispielhaften Ausführungsform eines erfindungsgemäßen computerimplementierten Verfahrens zur Materialdekomposition beziehungsweise eines entsprechenden computerimplementierten Trainingsverfahrens ist in FIG 3 dargestellt.

Ein Dekompositionsmodul 10, welches eine erste Funktion, beispielsweise ein erstes KNN, enthält, wird auf die erste rekonstruierte Volumenrekonstruktion 11 und die zweite rekonstruierte Volumenrekonstruktion 12 angewendet und liefert basierend darauf wenigstens eine materialspezifische Volumenrekonstruktion 13, 14, beispielsweise ein Jodrekonstruktion 14 und eine VNC-Rekonstruktion 13. Im Anwendungsfall ist die erste Funktion bereits trainiert und das computerimplementierte Verfahren damit beispielsweise abgeschlossen.

Während des Trainings der ersten Funktion wird eine Verlustfunktion abhängig von der wenigstens einen materialspezifischen Volumenrekonstruktion 13, 14 und wenigstens einer materialspezifischen Ground-Truth-Volumenrekonstruktion 15, 16 ausgewertet und Parameter der ersten Funktion werden abhängig von einem Ergebnis der Auswertung der Verlustfunktion durch ein Optimierungsmodul 17 aktualisiert.

Insbesondere wird ein erster Verlustterm L1 abhängig von der VNC-Rekonstruktion 13 und der zugehörigen Ground-Truth-VNC-Rekonstruktion 15 ausgewertet und ein zweiter Verlustterm L2 abhängig von der Jodrekonstruktion 14 und der zugehörigen Ground-Truth-Jodrekonstruktion 16. Die Verlustterme L1, L2 können beispielsweise bekannte Verlustterme sein oder darauf basieren, wie etwa ein mittlerer quadratischer Fehler, ein mittlerer absoluter Fehler, ein mittlerer absoluter prozentualer Fehler, eine strukturelle Ähnlichkeitsindex, ein histogrammbasierter Verlust, und so weiter. Die Verlustfunktion kann beispielsweise einer Summe oder gewichteten Summe der Verlustterme L1, L2 entsprechen.

Das Optimierungsmodul 17 kann bekannte Verfahren zur Aktualisierung der Parameter der ersten Funktion verwenden, beispielsweise Backpropagation.

Das Verfahren kann anstelle der Volumenrekonstruktionen 11, 12 analog basierend auf entsprechenden Röntgenprojektionsbildern durchgeführt werden. Anstelle der wenigstens einen materialspezifischen Volumenrekonstruktion 13, 14 resultiert dann wenigstens ein materialspezifisches Röntgenprojektionsbild.

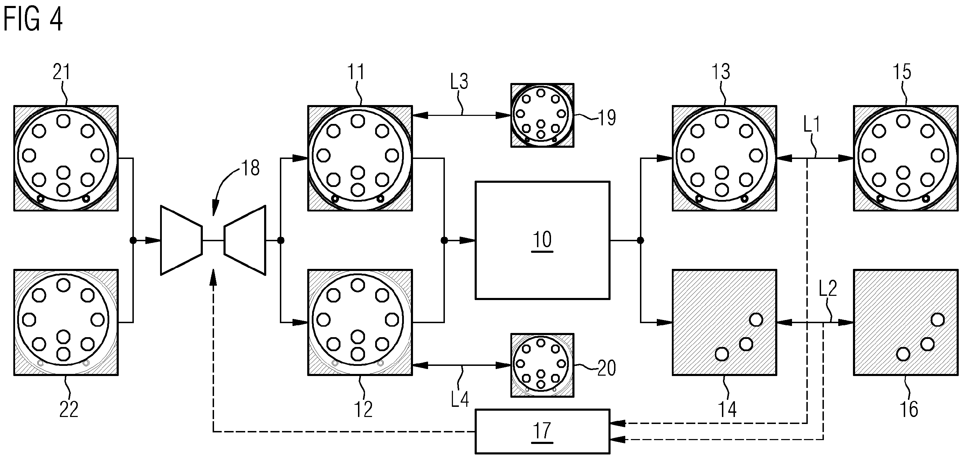

Eine schematische Darstellung einer weiteren beispielhaften Ausführungsform eines erfindungsgemäßen computerimplementierten Verfahrens zur Materialdekomposition beziehungsweise eines entsprechenden computerimplementierten Trainingsverfahrens, die auf der Ausführungsform der FIG 3 basieren, ist in FIG 4 dargestellt.

Hier handelt es sich bei den Volumenrekonstruktionen 11, 12 um artefaktreduzierte Volumenrekonstruktionen 11, 12. Dazu wird ein Artefaktreduktionsmodul, welches eine zweite Funktion 18, beispielsweise ein zweites KNN, enthält, auf ursprüngliche Volumenrekonstruktionen 21, 22 entsprechend dem ersten und dem zweiten Röntgenstrahlungsenergiespektrum angewendet und liefert die artefaktreduzierten Volumenrekonstruktionen 11, 12. Die restlichen Schritte entsprechen den bezüglich FIG 3 erläuterten. Im Anwendungsfall ist die zweite Funktion bereits trainiert und das computerimplementierte Verfahren damit beispielsweise abgeschlossen.

Während des Trainings der zweiten Funktion wird eine vorgegebene weitere Verlustfunktion ausgewertet. Die weitere Verlustfunktion enthält einen Verlustterm L3, welcher von der artefaktreduzierten ersten Volumenrekonstruktion 11 und einer artefaktreduzierten ersten Ground-Truth- Volumenrekonstruktion 19 abhängt, sowie einen weiteren Verlustterm L4, welcher von der prädizierten artefaktreduzierten zweiten Volumenrekonstruktion 12 und einer artefaktreduzierten zweiten Ground-Truth-Volumenrekonstruktion 20 abhängt. Parameter der zweiten Funktion 18 werden abhängig von einem Ergebnis der Auswertung der weiteren Verlustfunktion durch das Optimierungsmodul 17 aktualisiert.

Die Verlustterme L3, L4 können beispielsweise bekannte Verlustterme sein oder darauf basieren, wie etwa ein mittlerer quadratischer Fehler, ein mittlerer absoluter Fehler, ein mittlerer absoluter prozentualer Fehler, eine strukturelle Ähnlichkeitsindex, ein histogrammbasierter Verlust, und so weiter. Die weitere Verlustfunktion kann beispielsweise einer Summe oder gewichteten Summe der Verlustterme L3, L3 entsprechen.

Das Optimierungsmodul 17 kann bekannte Verfahren zur Aktualisierung der Parameter der ersten Funktion verwenden, beispielsweise Backpropagation. Das Optimierungsmodul 17 kann dabei insbesondere die erste Funktion und die zweite Funktion 18 getrennt voneinander trainieren.

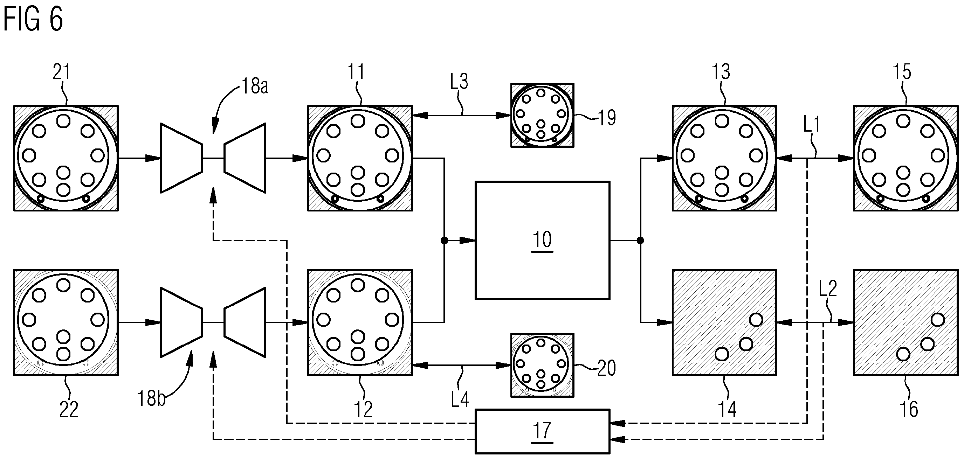

In manchen Ausführungsformen wird die zweite Funktion 18 durch eine zweite Funktion 18a, welche auf die ursprüngliche Volumenrekonstruktionen 21 angewendet wird und die artefaktreduzierte Volumenrekonstruktionen 11 liefert, und eine weitere zweite Funktion 18b, welche auf die ursprüngliche Volumenrekonstruktionen 22 angewendet wird und die artefaktreduzierte Volumenrekonstruktionen 12 liefert, ersetzt. Ein solches Verfahren ist schematisch in FIG 6 dargestellt.

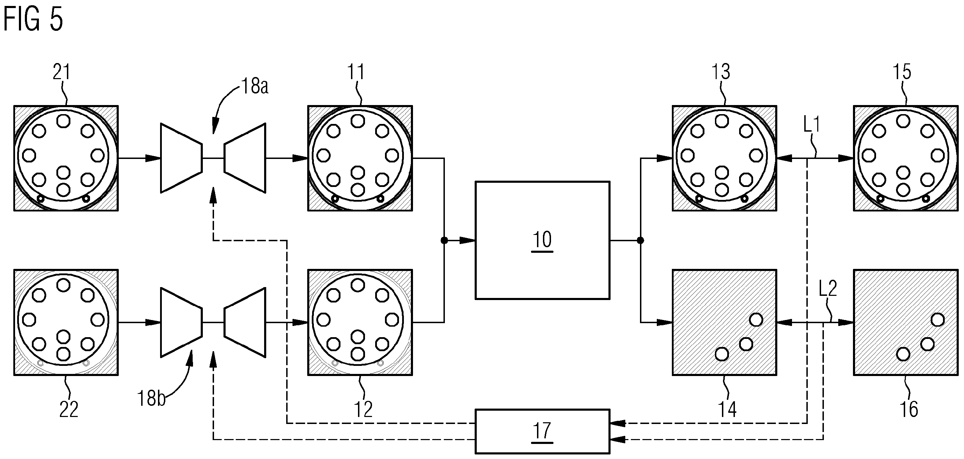

In alternativen Ausführungsformen wird auf die weitere Verlustfunktion verzichtet und die erste Funktion und die zweite Funktion 18 werden gemeinsam Ende-zu-Ende basierend auf der Verlustfunktion mit den Verlusttermen L1, L2 trainiert. Die Ground-Truth-Volumenrekonstruktionen 19, 20 sind dann nicht erforderlich. Auch solche Ausführungsformen lassen sich mit Ausführungsformen kombinieren, in denen die zweite Funktion 18 durch die zweiten Funktionen 18a, 18b ersetzt wird, wie schematisch in FIG 5 dargestellt.

Das Verfahren kann anstelle der rekonstruierten Volumina 11, 12 analog basierend auf entsprechenden Röntgenprojektionsbildern durchgeführt werden. Anstelle der wenigstens einen materialspezifischen Volumenrekonstruktion 13, 14 resultiert dann wenigstens ein materialspezifisches Röntgenprojektionsbild.

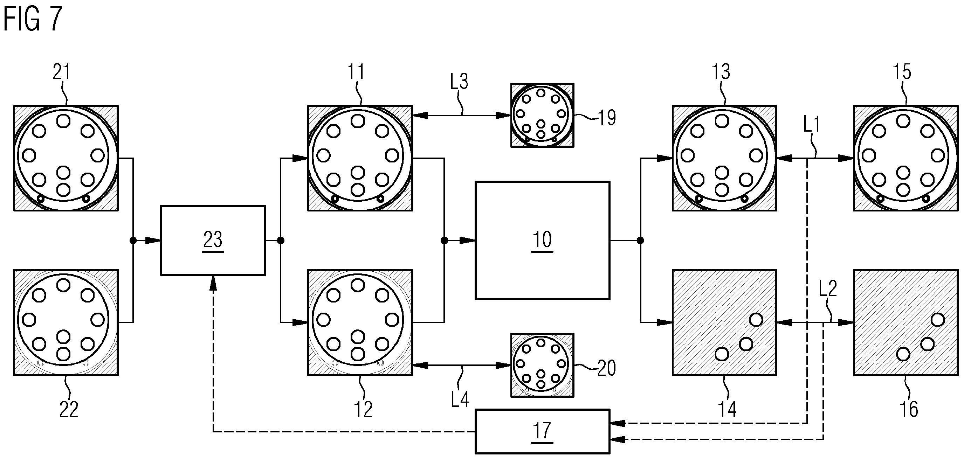

Eine schematische Darstellung einer weiteren beispielhaften Ausführungsform eines erfindungsgemäßen computerimplementierten Verfahrens zur Materialdekomposition beziehungsweise eines entsprechenden computerimplementierten Trainingsverfahrens, die auf der Ausführungsform der FIG 4 basieren, ist in FIG 7 dargestellt. In der Ausführungsform der FIG 7 ist die zweite Funktion 18 durch eine gemeinsame Filterfunktion 23, beispielsweise einen gemeinsamen bilateralen Filter ersetzt. Die Filterfunktion 23 ist nicht notwendigerweise als KNN ausgestaltet, ihre Parameter, beispielsweise Kernelgrößen und/oder eine Fenstergröße des Filters, können aber ebenfalls separat zur ersten Funktion basierend auf den Verlusttermen L3, L4, oder Ende-zu-Ende mit der ersten Funktion trainiert werden.

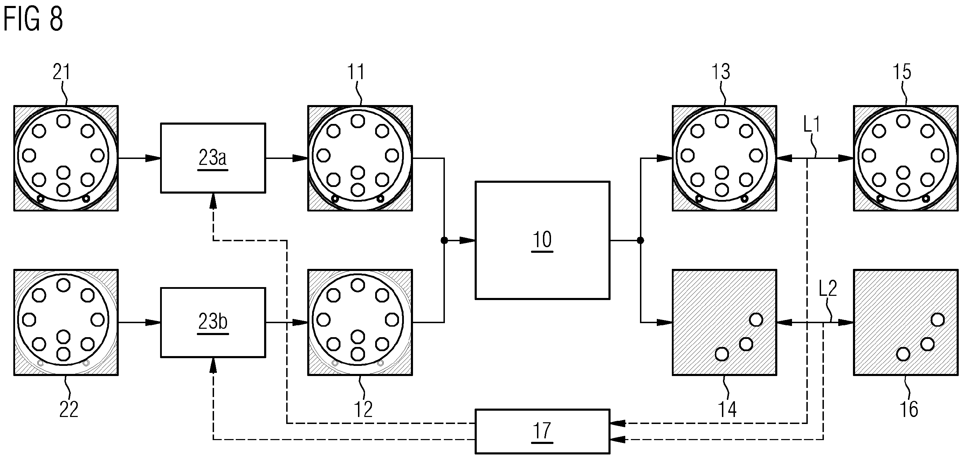

Eine schematische Darstellung einer weiteren beispielhaften Ausführungsform eines erfindungsgemäßen computerimplementierten Verfahrens zur Materialdekomposition beziehungsweise eines entsprechenden computerimplementierten Trainingsverfahrens, die auf der Ausführungsform der FIG 7 basiert, ist in FIG 8 dargestellt. Hier ist die gemeinsame Filterfunktion 23 durch zwei einzelne Filterfunktionen 23a, 23b ersetzt, die auf die ursprüngliche Volumenrekonstruktionen 21 beziehungsweise die ursprüngliche Volumenrekonstruktionen 22 angewendet werden. Das Training der Filterfunktionen 23a, 23b erfolgt Ende-zu-Ende mit der ersten Funktion wie bezüglich FIG 5 erläutert.

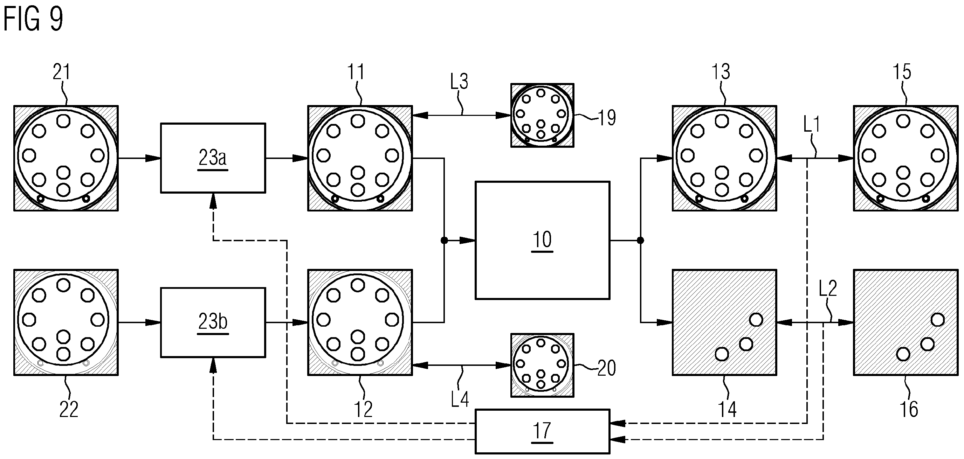

Eine schematische Darstellung einer weiteren beispielhaften Ausführungsform eines erfindungsgemäßen computerimplementierten Verfahrens zur Materialdekomposition beziehungsweise eines entsprechenden computerimplementierten Trainingsverfahrens, die auf der Ausführungsform der FIG 7 basiert, ist in FIG 9 dargestellt. Hier ist die gemeinsame Filterfunktion 23 durch zwei einzelne Filterfunktionen 23a, 23b ersetzt, die auf die ursprüngliche Volumenrekonstruktionen 21 beziehungsweise die ursprüngliche Volumenrekonstruktionen 22 angewendet werden. Das Training der Filterfunktionen 23a, 23b erfolgt separat zu der ersten Funktion, wie bezüglich FIG 6 erläutert.

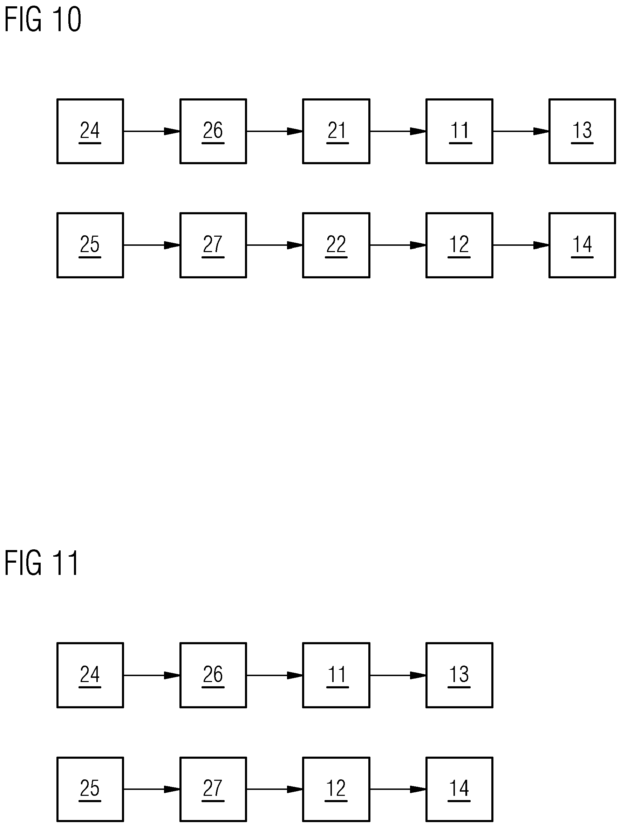

FIG 10 zeigt ein schematisches Ablaufdiagramm einer beispielhaften Ausführungsform eines erfindungsgemäßen Verfahrens zur Dual-Energie-Röntgenbildgebung unter Verwendung eines erfindungsgemäßen computerimplementierten Verfahrens zur Materialdekomposition.

Hierbei wird für das erste Röntgenstrahlungsenergiespektrum eine Vielzahl von ersten Röntgenprojektionsbildern 24 erzeugt. Basierend darauf wird eine Vielzahl von artefaktreduzierten und/oder gefilterten ersten Röntgenprojektionsbildern 26 erzeugt. Basierend darauf wird die erste Volumenrekonstruktion 21 erzeugt und wie oben bezüglich FIG 4 bis FIG 9 erläutert weiterverarbeitet.

Für das zweite Röntgenstrahlungsenergiespektrum wird eine Vielzahl von zweiten Röntgenprojektionsbildern 25 erzeugt. Basierend darauf wird eine Vielzahl von artefaktreduzierten und/oder gefilterten zweiten Röntgenprojektionsbildern 27 erzeugt. Basierend darauf wird die zweite Volumenrekonstruktion 22 erzeugt und wie oben bezüglich FIG 4 bis FIG 9 erläutert weiterverarbeitet.

FIG 11 zeigt ein schematisches Ablaufdiagramm einer weiteren beispielhaften Ausführungsform eines erfindungsgemäßen Verfahrens zur Dual-Energie-Röntgenbildgebung unter Verwendung eines erfindungsgemäßen computerimplementierten Verfahrens zur Materialdekomposition.

Hierbei wird für das erste Röntgenstrahlungsenergiespektrum die Vielzahl von ersten Röntgenprojektionsbildern 24 erzeugt. Basierend darauf wird die Vielzahl von artefaktreduzierten und/oder gefilterten ersten Röntgenprojektionsbildern 26 erzeugt. Basierend darauf wird die erste Volumenrekonstruktion 11 erzeugt und wie oben bezüglich FIG 3 erläutert weiterverarbeitet.

Für das zweite Röntgenstrahlungsenergiespektrum wird die Vielzahl von zweiten Röntgenprojektionsbildern 25 erzeugt. Basierend darauf wird die Vielzahl von artefaktreduzierten und/oder gefilterten zweiten Röntgenprojektionsbildern 27 erzeugt. Basierend darauf wird die zweite Volumenrekonstruktion 12 erzeugt und wie oben bezüglich FIG 3 erläutert weiterverarbeitet.

Wie beschrieben, insbesondere unter Bezugnahme auf die Figuren, ermöglicht es die Erfindung, die Qualität von materialspezifischen Bilddaten bei der Materialdekomposition bei der Dual-Energie-Röntgenbildgebung zu verbessern, sodass insbesondere eine verbesserte quantitative Materialanalyse ermöglicht wird.

Unabhängig vom grammatikalischen Geschlecht eines bestimmten Begriffes sind Personen mit männlicher, weiblicher oder anderer Geschlechteridentität mit umfasst.

## Patentansprüche

1. Computerimplementiertes Verfahren zur Materialdekomposition bei der Dual-Energie-Röntgenbildgebung, wobei
- ein erster Röntgenbilddatensatz (21, 24) entsprechend einem ersten Röntgenstrahlungsenergiespektrum und ein zweiter Röntgenbilddatensatz (22, 25) entsprechend einem zweiten Röntgenstrahlungsenergiespektrums erhalten werden;
- durch Anwendung eines Dekompositionsmoduls (10), welches eine trainierte erste Funktion enthält, auf Eingangsdaten, welche von dem ersten Röntgenbilddatensatz (21, 24) und dem zweiten Röntgenbilddatensatz (22, 25) abhängen, wenigstens ein materialspezifischer Bilddatensatz (13, 14) erzeugt wird.

2. Computerimplementiertes Verfahren nach Anspruch 1, wobei
- der erste Röntgenbilddatensatz (21, 24) einem ersten Röntgenprojektionsbild entspricht und der zweite Röntgenbilddatensatz (22, 25) einem zweiten Röntgenprojektionsbild entspricht; oder
- der erste Röntgenbilddatensatz (21, 24) einem ersten rekonstruierten Volumen entspricht und der zweite Röntgenbilddatensatz (22, 25) einem zweiten rekonstruierten Volumen entspricht.

3. Computerimplementiertes Verfahren nach einem der vorhergehenden Ansprüche, wobei der wenigstens eine materialspezifischer Bilddatensatz (13) einen Kontrastmittelbilddatensatz (14) und/oder einen virtuellen Nicht-Kontrast-Bilddatensatz (13) beinhaltet.

4. Computerimplementiertes Verfahren nach einem der vorhergehenden Ansprüche, wobei
- durch Anwendung eines Artefaktreduktionsmoduls, welches wenigstens eine trainierte zweite Funktion (18, 18a, 18b) enthält, auf weitere Eingangsdaten, welche von dem ersten Röntgenbilddatensatz (21, 24) und dem zweiten Röntgenbilddatensatz (22, 25) abhängen, ein artefaktreduzierter erster Röntgenbilddatensatz (11) und artefaktreduzierter zweiter Röntgenbilddatensatz (12) erzeugt werden; und
- die Eingangsdaten von dem artefaktreduzierten ersten Röntgenbilddatensatz (11) und dem artefaktreduzierten zweiten Röntgenbilddatensatz (12) abhängen.

5. Computerimplementiertes Verfahren nach einem der Ansprüche 1 bis 3, wobei
- durch Anwendung eines Filtermoduls, welches wenigstens eine Filterfunktion (23, 23a, 23b) enthält, auf weitere Eingangsdaten, welche von dem ersten Röntgenbilddatensatz (21, 24) und dem zweiten Röntgenbilddatensatz (22, 25) abhängen, ein gefilterter erster Röntgenbilddatensatz und ein gefilterter zweiter Röntgenbilddatensatz (22, 25) erzeugt werden; und
- die Eingangsdaten von dem gefilterten ersten Röntgenbilddatensatz und dem gefilterten zweiten Röntgenbilddatensatz abhängen.

6. Computerimplementiertes Verfahren nach Anspruch 5, wobei die wenigstens eine Filterfunktion (23, 23a, 23b) wenigstens eine Filterfunktion (23, 23a, 23b) zur bilateralen Filterung und/oder wenigstens eine Filterfunktion (23, 23a, 23b) zur differenzierbaren geführten Filterung enthält.

7. Verfahren zur Dual-Energie-Röntgenbildgebung, wobei
- ein erster Röntgenbilddatensatz (21, 24), welcher ein abzubildendes Objekt (5) darstellt, erzeugt wird, indem erste Röntgenstrahlung entsprechend einem ersten Röntgenstrahlungsenergiespektrum erzeugt wird und durch das Objekt (5) hindurchtretende Anteile der ersten Röntgenstrahlung detektiert werden;
- ein zweiter Röntgenbilddatensatz (22, 25), welcher das Objekt(5) darstellt, erzeugt wird, indem zweite Röntgenstrahlung entsprechend einem zweiten Röntgenstrahlungsenergiespektrum erzeugt wird und durch das Objekt(5) hindurchtretende Anteile der ersten Röntgenstrahlung detektiert werden;
- ein computerimplementiertes Verfahren nach einem der vorhergehenden Ansprüche durchgeführt wird.

8. Verfahren nach Anspruch 7, wobei das Verfahren als Computertomographieverfahren oder als Kegelstrahlcomputertomographieverfahren durchgeführt wird und
- der erste Röntgenbilddatensatz (21, 24) einem ersten Röntgenprojektionsbild entspricht, der zweiten Röntgenbilddatensatz (22, 25) einem zweiten Röntgenprojektionsbild entspricht, wenigstens ein rekonstruiertes Volumen wird abhängig von dem wenigstens einen materialspezifischen Bilddatensatz (13, 14) erzeugt wird; oder
- der erste Röntgenbilddatensatz (21, 24) einem ersten rekonstruierten Volumen entspricht und der zweite Röntgenbilddatensatz (22, 25) einem zweiten rekonstruierten Volumen entspricht.

9. Computerimplementiertes Trainingsverfahren zur Bereitstellung einer trainierten ersten Funktion zur Verwendung in einem computerimplementierten Verfahren nach einem der Ansprüche 1 bis 6, wobei
- ein erster Röntgentrainingsbilddatensatz entsprechend einem ersten Röntgenstrahlungsenergiespektrum und ein zweiter Röntgentrainingsbilddatensatz entsprechend einem zweiten Röntgenstrahlungsenergiespektrum erhalten werden;
- wenigstens ein materialspezifischer Ground-Truth-Bilddatensatz (15, 16) für den ersten Röntgentrainingsbilddatensatz und den zweiten Röntgentrainingsbilddatensatz erhalten wird;
- durch Anwendung eines Dekompositionsmoduls (10), welches die untrainierte oder teilweise trainierte erste Funktion enthält, auf Eingangstrainingsdaten, welche von dem ersten Röntgentrainingsbilddatensatz und dem zweiten Röntgentrainingsbilddatensatz abhängen, wenigstens ein prädizierter materialspezifischer Bilddatensatz (13, 14) erzeugt wird;
- eine vorgegebene Verlustfunktion (L1, L2) ausgewertet wird, die von dem wenigstens einen prädizierten materialspezifischen Bilddatensatz (13, 14) und dem wenigstens einen materialspezifischen Ground-Truth-Bilddatensatz (15, 16) abhängt; und
- Parameter der ersten Funktion abhängig von einem Ergebnis der Auswertung der Verlustfunktion (L1, L2) aktualisiert werden.

10. Computerimplementiertes Trainingsverfahren zur Bereitstellung einer trainierten ersten Funktion und wenigstens einer trainierten zweiten Funktion (18, 18a, 18b) zur Verwendung in einem computerimplementierten Verfahren nach Anspruch 4, wobei
- ein computerimplementiertes Trainingsverfahren nach Anspruch 9 durchgeführt wird;
- durch Anwendung eines Artefaktreduktionsmoduls, welches die wenigstens eine untrainierte oder teilweise trainierte zweite Funktion (18, 18a, 18b) enthält, auf weitere Eingangstrainingsdaten, welche von dem ersten Röntgentrainingsbilddatensatz und dem zweiten Röntgentrainingsbilddatensatz abhängen, ein prädizierter artefaktreduzierter erster Röntgenbilddatensatz (21, 24) und ein prädizierter artefaktreduzierter zweiter Röntgenbilddatensatz (22, 25) erzeugt werden; und
- die Eingangstrainingsdaten von dem prädizierten artefaktreduzierten ersten Röntgenbilddatensatz (21, 24) und dem prädizierten artefaktreduzierten zweiten Röntgenbilddatensatz (22, 25) abhängen; und
- Parameter der wenigstens einen zweiten Funktion (18, 18a, 18b) abhängig von einem Ergebnis der Auswertung der Verlustfunktion (L1, L2) aktualisiert werden.

11. Computerimplementiertes Trainingsverfahren zur Bereitstellung einer trainierten ersten Funktion und wenigstens einer trainierten zweiten Funktion (18, 18a, 18b) zur Verwendung in einem computerimplementierten Verfahren nach Anspruch 4, wobei
- ein computerimplementiertes Trainingsverfahren nach Anspruch 9 durchgeführt wird;
- ein artefaktreduzierter erster Ground-Truth-Röntgenbilddatensatz (19) für den ersten Röntgentrainingsbilddatensatz und ein artefaktreduzierter zweiter Ground-Truth-Röntgenbilddatensatz (20) für den zweiten Röntgentrainingsbilddatensatz erhalten werden;
- durch Anwendung eines Artefaktreduktionsmoduls, welches die wenigstens eine untrainierte oder teilweise trainierte zweite Funktion (18, 18a, 18b) enthält, auf weitere Eingangstrainingsdaten, welche von dem ersten Röntgentrainingsbilddatensatz und dem zweiten Röntgentrainingsbilddatensatz abhängen, ein prädizierter artefaktreduzierter erster Röntgenbilddatensatz und ein prädizierter artefaktreduzierter zweiter Röntgenbilddatensatz erzeugt werden; und
- die Eingangstrainingsdaten von dem prädizierten artefaktreduzierten ersten Röntgenbilddatensatz und dem prädizierten artefaktreduzierten zweiten Röntgenbilddatensatz abhängen;
- eine vorgegebene weitere Verlustfunktion (L3, L4) ausgewertet wird, die einen Verlustterm (L3), welcher von dem prädizierten artefaktreduzierten ersten Röntgenbilddatensatz und dem artefaktreduzierten ersten Ground-Truth-Röntgenbilddatensatz (19) abhängt, sowie einen weiteren Verlustterm (L4), welcher von dem prädizierten artefaktreduzierten zweiten Röntgenbilddatensatz und dem artefaktreduzierten zweiten Ground-Truth-Röntgenbilddatensatz (20) abhängt, enthält; und
- Parameter der wenigstens einen zweiten Funktion (18, 18a, 18b) abhängig von einem Ergebnis der Auswertung der weiteren Verlustfunktion (L3, L4) aktualisiert werden.

12. Computerimplementiertes Trainingsverfahren zur Bereitstellung einer trainierten ersten Funktion und wenigstens einer Filterfunktion (23, 23a, 23b) zur Verwendung in einem computerimplementierten Verfahren nach einem der Ansprüche 5 oder 6, wobei
- ein computerimplementiertes Trainingsverfahren nach Anspruch 9 durchgeführt wird;
- ein gefilterter erster Ground-Truth-Röntgenbilddatensatz für den ersten Röntgentrainingsbilddatensatz und ein gefilterter zweiter Ground-Truth-Röntgenbilddatensatz für den zweiten Röntgentrainingsbilddatensatz erhalten werden;
- durch Anwendung eines Filtermoduls, welches die wenigstens eine Filterfunktion (23, 23a, 23b) enthält, auf weitere Eingangstrainingsdaten, welche von dem ersten Röntgentrainingsbilddatensatz und dem zweiten Röntgentrainingsbilddatensatz abhängen, ein prädizierter gefilterter erster Röntgenbilddatensatz (21, 24) und ein prädizierter gefilterter zweiter Röntgenbilddatensatz (22, 25) erzeugt werden; und
- die Eingangstrainingsdaten von dem prädizierten gefilterten ersten Röntgenbilddatensatz (21, 24) und dem prädizierten gefilterten zweiten Röntgenbilddatensatz (22, 25) abhängen;
- eine vorgegebene weitere Verlustfunktion (L3, L4) ausgewertet wird, die einen Verlustterm (L3), welcher von dem prädizierten gefilterten ersten Röntgenbilddatensatz (21, 24) und dem gefilterten ersten Ground-Truth-Röntgenbilddatensatz abhängt, sowie einen weiteren Verlustterm (L4), welcher von dem prädizierten gefilterten zweiten Röntgenbilddatensatz (22, 25) und dem gefilterten zweiten Ground-Truth-Röntgenbilddatensatz abhängt, enthält; und
- Parameter der wenigstens einen Filterfunktion (23, 23a, 23b) abhängig von einem Ergebnis der Auswertung der weiteren Verlustfunktion (L3, L4) aktualisiert werden.

13. Datenverarbeitungsvorrichtung (2) aufweisend wenigstens eine Recheneinheit, die dazu angepasst ist, ein computerimplementiertes Verfahren nach einem der Ansprüche 1 bis 6 und/oder ein computerimplementiertes Trainingsverfahren nach einem der Ansprüche 8 bis 12 durchzuführen.

14. Röntgenbildgebungsvorrichtung (1) aufweisend eine Datenverarbeitungsvorrichtung (2) nach Anspruch 13, eine Röntgenquelle (4), einen Röntgendetektor (3) und wenigstens eine Steuereinheit wobei
- die wenigstens eine Steuereinheit dazu eingerichtet ist, die Röntgenquelle (4) anzusteuern, erste Röntgenstrahlung entsprechend einem ersten Röntgenstrahlungsenergiespektrum zu erzeugen;
- der Röntgendetektor (3) dazu eingerichtet ist, den ersten Röntgenbilddatensatz (21, 24), welcher ein abzubildendes Objekt (5) darstellt, zu erzeugen und dazu durch das Objekt (5) hindurchtretende Anteile der ersten Röntgenstrahlung zu detektieren;
- die wenigstens eine Steuereinheit dazu eingerichtet ist, die Röntgenquelle (4) anzusteuern, zweite Röntgenstrahlung entsprechend einem zweiten Röntgenstrahlungsenergiespektrum zu erzeugen; und
- der Röntgendetektor (3) dazu eingerichtet ist, den zweiten Röntgenbilddatensatz (22, 25), welcher das Objekt (5) darstellt, zu erzeugen und dazu durch das Objekt (5) hindurchtretende Anteile der zweiten Röntgenstrahlung zu detektieren.

15. Computerprogrammprodukt aufweisend
- Befehle, die bei Ausführung durch eine Datenverarbeitungsvorrichtung (2) die Datenverarbeitungsvorrichtung (2) dazu veranlassen, ein computerimplementiertes Verfahren nach einem der Ansprüche 1 bis 6 und/oder ein computerimplementiertes Trainingsverfahren nach einem der Ansprüche 8 bis 12 durchzuführen; und/oder
- weitere Befehle, die bei Ausführung durch eine Röntgenbildgebungsvorrichtung (1) nach Anspruch 14 die Röntgenbildgebungsvorrichtung (1) dazu veranlassen, ein Verfahren nach einem der Ansprüche 7 oder 8 durchzuführen.
